(19) **Europäisches Patentamt**
**European Patent Office**
**Office européen des brevets**

(11) **EP 4 613 198 A1**

(12) **EUROPEAN PATENT APPLICATION**

(43) Date of publication:
**10.09.2025 Bulletin 2025/37**

(21) Application number: **25157920.7**

(22) Date of filing: **14.02.2025**

(51) International Patent Classification (IPC):
*A61B 5/18* (2006.01)   *A61B 5/369* (2021.01)
*A61B 5/389* (2021.01)   *A61B 5/398* (2021.01)
*A61B 5/00* (2006.01)

(52) Cooperative Patent Classification (CPC):
**A61B 5/18; A61B 5/369; A61B 5/389; A61B 5/398;**
**A61B 5/4809; A61B 5/7264; A61B 5/7267**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **04.03.2024 CN 202410240626**

(71) Applicant: **Robert Bosch GmbH**
**70442 Stuttgart (DE)**

(72) Inventors:
• **XIA, Lijuan**
 **Shanghai, 200335 (CN)**
• **ZHANG, Shouyi**
 **Shanghai, 200335 (CN)**
• **YU, Yifei**
 **Shanghai, 200335 (CN)**
• **SUN, Zhigang**
 **Shanghai, 200335 (CN)**

(74) Representative: **Isarpatent**
**Patent- und Rechtsanwälte Partg mbB**
**Friedrichstraße 31**
**80801 München (DE)**

(54) **METHODS AND DEVICES FOR ESTIMATING HUMAN ALERTNESS**

(57)    The present invention provides a method for estimating the alertness of the human body. The method comprises: Obtaining a bioelectric signal from the human body; obtaining feature data for estimating the alertness of the human body based on the bioelectric signal; and estimating the alertness value of the human body using a regression neural network based on the feature data of the bioelectric signal.

FIG. 2

## Description

### Technical Field

The present invention is generally related to techniques for detecting human alertness, and more particularly to techniques for detecting human alertness based on bioelectric signals of the human body using deep machine learning models.

### Background Art

In people's daily lives and production operations, physical fatigue or inattention can lead to significant safety hazards, resulting in loss of life and property. Data show that more than 1 million people worldwide have lost their lives as a direct result of traffic accidents caused by driver fatigue. According to Chinese railway system accident statistics, accidents occurring between 02:00 and 05:00 a.m. account for 60% of major accidents caused by driver fatigue. In addition, excessive worker fatigue can also cause significant safety incidents in some highpressure, high-altitude, and other hazardous environmental operations. This is due to the inattention, slow reactions, and inaccurate judgments that occur when the human body is operating under fatigue, resulting in accidents due to operational errors. Therefore, monitoring the level of fatigue of the human body in some scenarios is important for safe living and production.

Currently, it is a common practice to detect whether the human body is in a state of fatigue based on the brain electrical signals (e.g., EEG) of the human body. This detection method based on the physiological signals of the human body has advantages including strong objectivity and high accuracy. With the development of artificial intelligence technology and the improvement of computer processing capabilities, there have also been many approaches in recent years that have utilized traditional or deep machine learning models to judge whether the human body is in a state of fatigue based on the EEG signals of the human body. However, current machine learning methods usually use the detection of the state of fatigue of the human body as a classification task, using existing convolutional neural networks (CNN) or recurrent neural networks (RNN) to predict the state of fatigue of the human body based on the EEG signals of the human body. This detection method is less accurate, e.g., the output result may simply be fatigued or not fatigued. Moreover, this approach may not be able to make accurate and reliable judgments in certain states of the human body. For example, a classification model is likely to make a judgment of not fatigued when the human body is already on the edge of being in or has just entered a fatigued state, resulting in potential safety hazards. Therefore, there remains a need for an improved technique for detecting the degree of fatigue of the human body.

### Summary of Invention

A brief description of one or more aspects according to the present disclosure is provided below in order to provide a basic understanding of these aspects. The present disclosure is not a broad overview of all aspects, nor is it intended to identify the key elements of all aspects or delineate the scope of any or all aspects. Its sole purpose is to present some concepts of one or more aspects in a simplified form as a preamble to the more detailed description that follows.

According to one aspect of the present disclosure, a method is provided for estimating the alertness of the human body. The method comprises: Obtaining a bioelectric signal from the human body; obtaining feature data for estimating the alertness of the human body based on the bioelectric signal; and estimating the alertness value of the human body using a regression neural network based on the feature data of the bioelectric signal.

According to one aspect of the present disclosure, a device is provided for estimating the alertness of the human body. The device comprises: a signal acquisition module for acquiring a bioelectric signal of the human body; a feature extraction module for obtaining feature data for estimating the alertness of the human body based on the bioelectric signal; and a regression neural network for estimating the alertness value of the human body based on the feature data of the bioelectric signal.

According to another aspect of the present disclosure, a device is provided for estimating the alertness of the human body. The device may comprise a memory and at least one processor coupled to the memory. The at least one processor may be configured to: Obtain a bioelectric signal from the human body; obtain feature data for estimating the alertness of the human body based on the bioelectric signal; and estimate the alertness value of the human body using a regression neural network based on the feature data of the bioelectric signal.

According to another aspect of the present disclosure, a computer-readable medium is provided that stores computer program code for estimating the alertness of the human body. The computer program code, when executed by the processor, may cause the processor to obtain a bioelectric signal from the human body; obtain feature data for estimating the alertness of the human body based on the bioelectric signal; and estimate the alertness value of the human body using a regression neural network based on the feature data of the bioelectric signal.

According to another aspect of the present disclosure, a computer program product is provided for estimating the alertness of the human body. The computer program product may comprise processor-executable computer program code. The processor-executable computer program code is used for: Obtaining a bioelectric signal from the human body; obtaining feature data for estimating the alertness of the human body based on the bioelectric signal; and estimating the alertness value of the human body using a regression neural network based on the feature data of the bioelectric signal.

It should be noted that the above one or more aspects include the following detailed description and features that are specifically recorded in the Claims. The following specification and accompanying drawings detail some of the exemplary features from a variety of aspects. These features merely indicate a variety of ways in which the principles of various aspects may be implemented, and the present disclosure is intended to include all of these aspects and their equivalents.

**Description of Accompanying Drawings**

The following drawings describe various examples of the present disclosure for illustrative purposes only. Those skilled in the art will readily recognize from the following description that alternative examples of the methods and structures disclosed herein may be achieved without departing from the spirit and principles disclosed herein.

FIG. 1 illustrates a schematic diagram of an EEG signal for estimating the alertness of the human body according to one example of the present disclosure.
FIG. 2 illustrates a flowchart of a method for estimating the alertness of the human body according to one example of the present disclosure.
FIG. 3 illustrates a structural schematic diagram of a regression neural network for estimating the alertness of the human body according to one example of the present disclosure.
FIG. 4 illustrates a structural schematic diagram of a multihead attention module in a regression neural network according to one example of the present disclosure.
FIG. 5 illustrates a structural schematic diagram of an attention mechanism in a multihead attention module in a regression neural network according to one example of the present disclosure.
FIG. 6 illustrates a structural schematic diagram of a feedforward neural network in a regression neural network according to one example of the present disclosure.
FIG. 7 illustrates a block diagram of a device for estimating the alertness of the human body according to one example of the present disclosure.
FIG. 8 illustrates a block diagram of a device for estimating the alertness of the human body according to one example of the present disclosure.

**Specific Embodiments**

In the following description, numerous specific details are set forth to provide a thorough understanding of the examples of the present invention. However, those skilled in the relevant art will recognize that the invention can be practiced without one or more of the specific details, or by using alternative methods, components, etc., to practice the invention. In some instances, well-known structures and operations are not shown or described in detail to avoid unnecessarily obscuring the present invention.

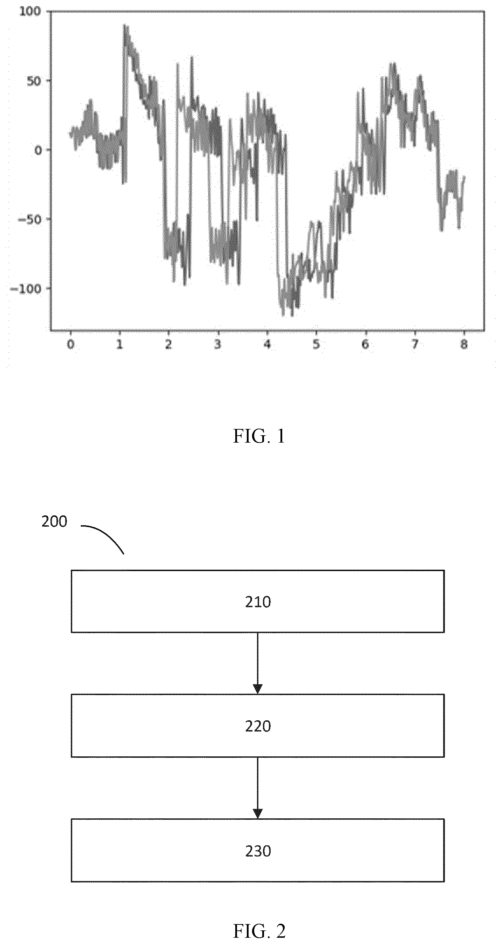

FIG. 1 illustrates a schematic diagram of an EEG signal for estimating the alertness of the human body according to one example of the present disclosure. Electroencephalography (EEG) signals generally refer to human scalp EEG, which is a map of the relationship between scalp potential difference and time. EEG signals directly reflect the electrical activity of brain tissue and can be used to assess the alertness of the human body. Alertness is associated with, among other things, the degree of fatigue and the degree of mental concentration of the human body, i.e., the degree of alertness may also reflect the degree of fatigue and/or mental concentration etc. of the human body. The devices used to record EEGs have developed from the original 1 or 2 channels to the now commonly used 16, 32, or 64 channels, and can even collect EEG signals from 512 scalp electrodes. The present disclosure is described and illustrated with EEG signals collected by forehead electrodes as an example, but the content of the present disclosure is not limited to the specific EEG signal collection method.

The vertical axis shown in FIG. 1 is the potential difference of the EEG signal in microvolts ($\mu$V). As can be seen from FIG. 1, the potential difference in the EEG signal is very low, typically less than 100'$\mu$V, and it is therefore susceptible to interference from surrounding electromagnetic field radiation and the electronic noise within the instrument during the acquisition process. The horizontal axis of FIG. 1 is the time axis of the EEG signal in seconds (s). FIG. 1 shows the EEG signal plot within an 8 s time window. In this example, the alertness of the human body from which the EEG signal was collected during the time period may be estimated based on the EEG signal within the 8 s time window. In other examples, the length of the time window may also be other values between 4 s and 12 s and may even be a length of time less than 4 s or greater than 12 s. The length of the time window may be determined based on the specific application scenario and design needs. For example, in scenarios where the results of the test need to be output quickly and frequently, the time window may be set to a shorter length; in scenarios where more accurate and reliable test results are needed, the time window may be set to a longer length.

In addition to EEG signals, bioelectric signals that reflect the physiological state of the human body, such as electrooculography signals (EOG) and electromyography signals (EMG), can also be used to estimate the alertness of the human body. For example, an EOG signal comprises signals that reflect the eye movement characteristics of the human body, such as eye movement, blinking frequency, etc., which can reflect the alertness of the human body. EMG signals can reflect the movement intent and muscle fatigue of the human body and are therefore also capable of being used to estimate the alertness of the human body. In various examples of the present disclosure, the alertness of the human body may be estimated based on various combinations of bioelectric signals, such as EEG signals, EOG signals, and EMG signals.

FIG. 2 illustrates a flowchart of a method 200 for estimating the alertness of the human body according to one example of the present disclosure. The method 200 may be performed by a device for estimating human alertness as described in the specific examples below or a wearable device having the functionality to estimate human alertness.

As shown in FIG. 2, in block 210, the method 200 can obtain a bioelectric signal from the human body. Obtaining a bioelectric signal from the human body may comprise obtaining a raw bioelectric signal capable of being used to estimate the alertness of the human body. The raw bioelectric signal may comprise at least one of the EEG signal, EOG signal, EMG signal, etc. of the human body as described above. For example, the method 200 can estimate the alertness of the human body based solely on the EEG signal of the human body. The method 200 can also estimate the alertness of the human body based on a combination of a variety of bioelectric signals of the human body, such as EEG and EOG signals. The method 200 may acquire a corresponding human bioelectric signal from existing bioelectric signal detection instruments or may utilize a bioelectric signal detection sensor integrated in a wearable device to acquire a bioelectric signal of the human body.

In block 210, obtaining a bioelectric signal of the human body may further comprise preprocessing an acquired raw bioelectric signal of the human body to at least partially remove noise and artifacts from the raw bioelectric signal. This is because bioelectric signals are very weak, typically at microvolt ($\mu$V) or millivolt (mV) magnitudes, and are therefore susceptible to interference from surrounding electromagnetic field radiation and electronic noise within the instrument during the acquisition process. A bandpass filter can be utilized to preprocess the raw bioelectric signal of the human body based on the characteristics of the various bioelectric signals. For example, the frequency of the EEG and EOG signals of the human body is typically in the range of 0 to 50 Hz, so a bandpass filter with a bandpass frequency of 1 Hz to 50 Hz can be utilized to preprocess the raw EEG and EOG signals of the human body to remove noise and artifacts from the raw signal. As another example, the frequency of the EMG signals of the human body is typically in the range of 0 to 500 Hz and the main energy concentration is in the frequency range of 20 to 150 Hz, so a bandpass filter with a bandpass frequency of 20 Hz to 150 Hz can be utilized to preprocess the raw EMG signal of the human body to remove noise and artifacts from the raw signal.

In block 220, the method 200 can obtain the feature data of a bioelectric signal for estimating the alertness of the human body based on an acquired bioelectric signal, e.g., a preprocessed bioelectric signal. In one example, the preprocessed bioelectric signal may be a bioelectric signal for a period of time after the preprocessing of a raw bioelectric signal within a time window as shown in Figure 1. That is, the raw bioelectric signal is acquired in units of time windows or the preprocessing operation in block 210 is processed in units of time windows. In another example, the preprocessed bioelectric signal may be a signal that is processed in real time and continuous in a temporal dimension. In block 220, the method 200 can intercept the preprocessed bioelectric signal in units of time windows to obtain a signal within a time period. The length of the time window can be 8 s or other values between 4 s to 12 s.

The method 200 can sample the preprocessed bioelectric signal within this time window to obtain a bioelectric signal data sequence, which can be expressed as $\{x_n\} := x_0, x_1, \cdots, x_{N-1}$, where N is the total number of sampling points, $x_n$ representing the potential difference value at each sampling point. The total number of sampling points N can be calculated based on the sampling frequency multiplied by the length of the time window. The sampling frequency may be determined based on the highest frequency of the bioelectric signal. For example, if the highest frequency of the preprocessed EEG signal is 50 Hz, the sampling frequency should be greater than or equal to twice 50 Hz, i.e., greater than or equal to 100 Hz. Accordingly, if the length of the time window is 6 s, the total number of sampling points should be greater than or equal to 600; if the length of the time window is 10 s, the total number of sampling points should be greater than or equal to 1000. In one example, the length of the time window is 8 s and the number of sampling points is 1000, i.e., the sampling frequency is 125 Hz.

Next, the sampled bioelectric signal data can be converted from the time domain to the frequency domain by a discrete Fourier transform to obtain the frequency domain sequence $\{X_k\} := X_0, X_1, \cdots, X_{K-1}$, as shown in the formula below, where K represents the number of sampling points (i.e., frequency points) in the frequency domain. The range of K can be any custom positive integer, e.g., the range of the frequency domain sequence index can be defined as [0, +2048], i.e., the value of $K$ is 2049, and then data of [1, 50] can be taken from it.

$$X_k = \sum_{n=0}^{N-1} x_n * e^{-\frac{i2\pi}{N}kn} = \sum_{n=0}^{N-1} x_n * \left[\cos\left(\frac{2\pi}{N}kn\right) - i * \sin\left(\frac{2\pi}{N}kn\right)\right]$$

The method 200 can obtain the feature data of a bioelectric signal for estimating the alertness of the human body based on the frequency domain sequence. The feature data may comprise at least one of the power spectral density (PSD) and the differential entropy (DE) of a bioelectric signal of the human body. In one example, both power spectral density and differential entropy may be employed as feature data. The power spectral density and differential entropy feature data can be sampled at a frequency range of 1 Hz to 50 Hz at a resolution of 2 Hz to obtain 25 pieces of power spectral density feature data and 25 pieces of differential entropy data, respectively, and the two are combined to form a vector with a dimension of 50 as the feature data of the bioelectric signal within the corresponding time window for estimating the alertness of the human body within that time window. In other examples, in different application scenarios, feature data of different dimensions may be constructed by employing different sampling frequencies and/or feature types depending on different design requirements, the computing capabilities of the device processor, etc. For example, in the above examples, in the event that two bioelectric signals, an EEG signal and an EOG signal, are detected simultaneously, a vector with a dimension of 100 can be obtained in a similar manner as feature data for the corresponding time window.

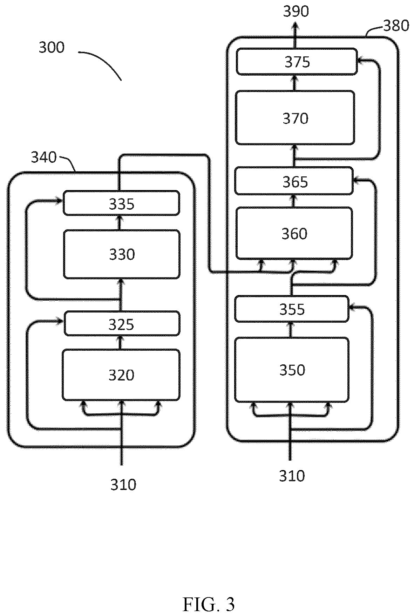

In block 230, the method 200 can utilize a regression neural network to estimate the alertness value of the human body based on the feature data of an acquired bioelectric signal. This alertness value is a specific value that is capable of representing the degree of alertness (e.g., degree of fatigue and/or degree of concentration) of the human body, which may be a natural number, an integer, a rational number, a real number, etc. The type and range of values of the alertness value depends on the type and range of values of the value labeled alertness in the data set used to train the regression neural network. The alertness values in the training data set may be manually labeled or may be automatically generated using computer vision processing techniques for facial expressions etc. In one example, the alertness value may be any rational number between 0 and 1. In another example, the alertness value may be any natural number between 1 and 100. The regression neural network for estimating the alertness value of the human body may be obtained from training based on existing network architectures for regression tasks or from training according to the neural network architecture of one example of the present disclosure described below in conjunction with FIGS. 3-6.

The method 200, after obtaining the estimated alertness value of the human body, may further judge whether the human body is in a state of fatigue or inattention based on the estimated alertness value and send a reminder signal to the human body or other supervisor when the human body is judged to be in a state of fatigue or inattention, e.g., by generating a vibration, issuing an alarm sound, sending a notification message to the supervisor, etc. The method 200 may compare the estimated alertness value to a predetermined threshold. When the estimated alertness value is above the threshold, the method 200 can judge that the human body is in good condition, i.e., not fatigued and focused. When the estimated alertness value is equal to or below the threshold, the method 200 can judge that the human body is in poor condition, e.g., fatigued or unfocused. By flexibly setting thresholds based on different application scenarios, the method 200 is able to make appropriate judgments in a variety of application scenarios. Higher thresholds may be set in scenarios with higher alertness requirements for the human body (e.g., in driving scenarios with higher vehicle speed) and lower thresholds may be set in scenarios with lower alertness requirements for the human body (e.g., in driving scenarios with lower vehicle speed).

FIG. 3 illustrates a structural schematic diagram of a regression neural network 300 for estimating the alertness of the human body according to one example of the present disclosure. The regression neural network 300 is a specific example of a neural network with an encoder-decoder architecture employing an attention mechanism. As shown in FIG. 3, the regression neural network 300 comprises an encoder 340 and a decoder 380. The encoder 340 may comprise a first multihead attention module 320, a first addition and normalization module 325, a first feedforward neural network 330, and a second addition and normalization module 335. The decoder 380 may comprise a second multihead attention module 350, a third addition and normalization module 355, a third multihead attention module 360 positioned behind the second multihead attention module 350, a fourth addition and normalization module 365, a second feedforward neural network 370, and a fifth addition and normalization module 375. Because collecting training data sets associated with the bioelectric signals of the human body is more costly and less data are collected compared to training data sets in other fields of deep learning, to improve the generalization capability of the trained regression neural network for better predictive performance, the regression neural network 300 comprises only one encoder and one decoder, thereby reducing network complexity.

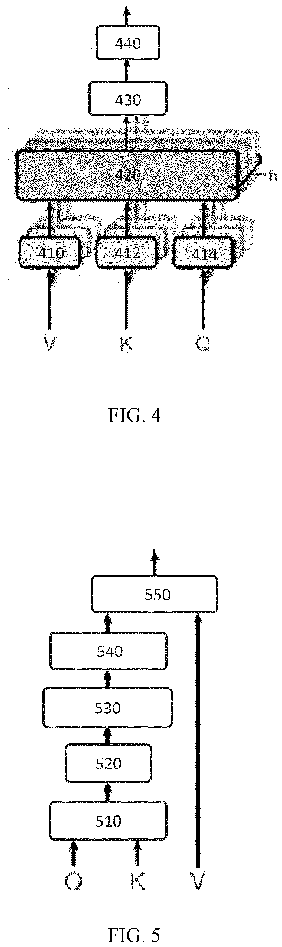

As shown in FIG. 3, the input 310 of the regression neural network 300 is provided to the first and second multihead attention modules 320 and 350. The method 200 may input the feature data of the bioelectric signal directly into the first and second multihead attention modules 320 and 350. FIG. 4 illustrates a structural schematic diagram of a multihead attention module in a regression neural network according to one example of the present disclosure. As shown in FIG. 4, the multihead attention modules may comprise multiple (h) parallel attention layers. In one example, the multihead attention modules in the regression neural network 300 may comprise the same number of attention layers (i.e., number of heads), for example, an 8-head attention module. In another example, the multihead attention module in the regression neural network 300 may comprise multihead attention modules with different numbers of heads. The data input to the multihead attention modules (e.g., bioelectric signal feature data comprising power spectral density and differential entropy) are respectively multiplied by the trained query matrix $M^Q$, the key matrix $M^K$, and the value matrix $M^V$ to obtain the query vector Q, the key vector K, and the value vector V, respectively. Th query vector Q, the key vector K, and the value vector V. Each head of the multihead attention module (i.e., each attention layer) is trained to have a different query matrix $M^Q$, key matrix $M^K$, and value matrix $M^V$ to assign attention weights to the input data from different spatial perspectives (e.g., based on different features).

The query vector Q, the key vector K, and the value vector V for each attention module head are processed into the attention layer 420 by the linear layers 410, 412, and 414, respectively. The attention layer 420 may assign different weights to each element of the input data vectors, resulting in output vectors with different attention weights. Since the input feature data include a bioelectric signal of the human body for a period of time within the detection time window, information at different time points and/or frequency points may be of different importance for estimating the alertness of the human body. For example, bioelectric signals collected at a moment of abrupt awakening are of lower importance to estimating the alertness of the human body when the human body is fatigued and sleepy; while when the human body is in a semi-fatigued state, bioelectric signals collected during a brief nap are of higher importance to estimating the alertness of the human body. By employing this attention mechanism in the regression neural network, the accuracy of estimating the alertness of the human body can be effectively improved. Each head of the multihead attention module may analyze the importance of each element in the input data vectors from different feature perspectives and stack the data vectors output by each attention layer 420 together at the bridge submodule 430 and output them to the next module or network after passing through the linear layer 440 again. In one example, the linear layer may be a fully connected neural network. In some examples, the multihead attention module may also not comprise a linear layer.

FIG. 5 illustrates a structural schematic diagram of an attention mechanism in a multihead attention module in a regression neural network according to one example of the present disclosure. As shown in FIG. 5, the attention mechanism adopted by each attention layer 420 comprises performing a dot product of the query vector Q and the key vector K (transposed) in the dot product submodule 510 and scaling (e.g., multiplying $\frac{1}{\sqrt{d_k}}$ ) the dot product result of the query vector Q and the key vector K according to the dimension $d_k$ of the query vector Q and the key vector K in the scaling submodule 520 to solve the problem of the gradient being too small during training.

In the masking submodule 530, fill masking or post-masking may be performed on the vector or matrix. Fill masking may be used to solve the problem of inconsistent dimensions of vectors or matrices. Post-masking can mask the data at subsequent time points when processing the current time point data. The masking submodule 530 is optional. For example, each of the attention layers of the first and third multihead attention modules 320 and 360 may not comprise a masking submodule; the second multihead attention module 350 may comprise a masking submodule for post-masking.

In the exponential normalization submodule 540, the softmax function can be utilized to process inputs (e.g., the product of the scaled query vector Q and the key vector K) and input the processing results into the dot product operation with the value vector V in the dot product submodule 550 to obtain the processing results for each attention layer. The function of each attention layer may be represented by the following formula:

$$\text{Attention}(Q, K, V) = \text{softmax}\left(\frac{QK^T}{\sqrt{d_k}}\right)V \text{。}$$

In one example, the output of the first multihead attention module 320 may be input to the first addition and normalization module 325. The input feature data vector 310 can be added to the vector output by the first multihead attention module 320 and normalized in the first addition and normalization module 325. This operation may add the input feature data to subsequent processing to avoid losing useful information in the input feature data after processing by the multihead attention module. In some examples, a plurality of input feature data vectors can be combined into a matrix form to perform matrix operations according to the formula above to improve execution speed. In this instance, the output of the multihead attention module may be a matrix. A broadcasting mechanism may be utilized in the first addition and normalization module 325 to perform an additive operation on a matrix of input feature data vectors 310 and output from the first multihead attention module 320. The processing results of the first addition and normalization module 325 may be input to the first feedforward neural network 330 and the second addition and normalization module 335.

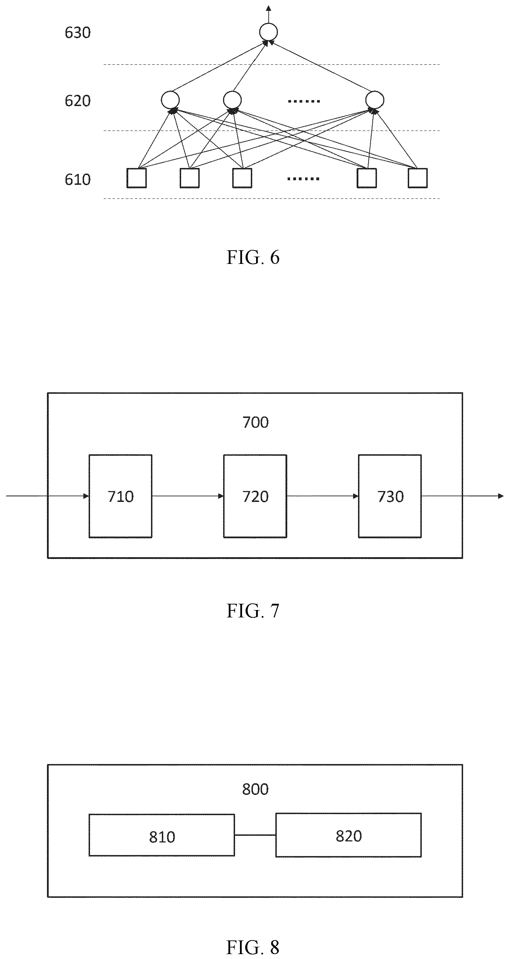

FIG. 6 illustrates a structural schematic diagram of a feedforward neural network in a regression neural network according to one example of the present disclosure. For example, the first feedforward neural network 330 may be a fully connected neural network that comprises an input layer 610, one or more hidden layers 620, and an output layer 630. The number of neuron units in the input layer 10 depends on the dimension of the vector input into the feedforward neural network. In one example, the first feedforward neural network 330 may comprise only one hidden layer and only one output neuron unit in the output layer 630 in order to reduce network complexity to obtain a regression neural network with generalization capability using a limited training data set. The number of hidden layers 620 and the number of neurons in each of the hidden layers can also be determined as hyperparameters during the training process.

The processing result of the first feedforward neural network 330 may be input into the second addition and normalization module 335. Operations similar to those of the first addition and normalization module 325 may be performed in the second addition and normalization module 335. The output of the second addition and normalization module 335 may be provided to the third multihead attention module 360 in the decoder 380 for calculating the query vector Q and the key vector K. The third multihead attention module 360 may calculate the value vector V based on the output of the second multihead attention module 350 and the third addition and normalization module 355. The second multihead attention module 350 and the third addition and normalization module 355 may perform similar processing operations to the first multihead attention module 320 and the first addition and normalization module 325 based on the input feature data. In one example, the second multihead attention module 350 may comprise a masking operation. The processing results of the second multihead attention module 350 and the third addition and normalization module 355 may also be input to the fourth addition and normalization module 365.

In the third multihead attention module 360, processing according to the attention mechanism is performed for the query vector Q and the key vector K calculated based on the output of the second addition and normalization module 335 and the value vector V calculated based on the output of the third addition and normalization module 355. For the specific processing, refer to the description in conjunction with FIGS. 4 and 5. The fourth addition and normalization module 365 may perform similar processing to the other addition and normalization modules (e.g., 325, 335, and 355).

The processing results of the fourth addition and normalization module 365 may be input to the second feedforward neural network 370 and the fifth addition and normalization module 375. The second feedforward neural network 370 may have a similar architecture to the first feedforward neural network 330 and perform similar processing. For specifics, refer to the description in conjunction with FIG. 6. The fifth addition and normalization module 375 may perform similar processing to the other addition and normalization modules (e.g., 325, 335, 355, and 365). The similar processing referred to herein refers merely to processing procedures and functions being similar, but different modules and neural networks will be trained with different parameters.

The output 390 of the fifth addition and normalization module 375 may be the estimated alertness value of the human body based on the feature data of the input bioelectric signal. Although not shown in FIG. 3, in another example, the output 390 of the fifth addition and normalization module 375 may further be processed through a linear layer to obtain the alertness value of the human body. The regression neural network described above in conjunction with FIGS. 3-6 may be used by the method 200 to estimate the alertness value of the human body based on the feature data of the bioelectric signal of the human body.

FIG. 7 illustrates a block diagram of a device 700 for estimating the alertness of the human body according to one example of the present disclosure. The device 700 may be a wearable device (e.g., a helmet, eyewear, watch, etc.) with the function of estimating the alertness of the human body or may be a dedicated device for estimating the alertness of the human body that can be installed on a wearable device, vehicle, etc. As shown in FIG. 7, the device 700 may comprise a signal acquisition module 710, a feature extraction module 720, and a regression neural network 730. Further, the device 700 may further comprise a bioelectric signal collection module, an early warning module, etc.

The signal acquisition module 710 may be used to obtain a bioelectric signal from the human body. The signal acquisition module 710 may obtain a raw bioelectric signal of the human body, e.g., at least one of a variety of bioelectric signals associated with the alertness of the human body, such as an EEG signal, an EOG signal, an EMG signal, etc. In one example, the signal acquisition module 710 may receive one or more bioelectric signals of the human body from outside the device 700. In another example, the signal acquisition module 700 may directly obtain a bioelectric signal of the human body from a bioelectric signal collection module integrated within the device 700 (e.g., a sensor for collecting a bioelectric signal of the human body). The signal acquisition module 710 may also preprocess the acquired raw bioelectric signal of the human body to at least partially remove noise and artifacts from the raw bioelectric signal. For example, the signal acquisition module 710 may comprise a bandpass filter for filtering the raw bioelectric signal of the human body. The bandpass filter may have different bandpass frequencies depending on the spectral characteristics of the different bioelectric signals to be processed. For example, the bandpass frequency of the bandpass filter may be between 1 Hz and 50 Hz for an EEG or EOG signal of the human body. The signal acquisition module 710 may provide an acquired human bioelectric signal, such as a preprocessed bioelectric signal, to the feature extraction module 720.

The feature extraction module 720 may obtain the feature data of a bioelectric signal for estimating the alertness of the human body based on a bioelectric signal of the human body, e.g., a preprocessed bioelectric signal. The feature extraction module 720 may comprise a Fourier transform submodule, such as a DFT or FFT, for example, to transform a bioelectric signal within a time window (e.g., 8 seconds) from the time domain to the frequency domain. The feature extraction module 720 may comprise submodules for calculating power spectral density and/or differential entropy. The feature extraction module 720 may combine different types of feature data derived from the various submodules into a feature data vector and input it to the regression neural network 730.

The regression neural network 730 can estimate the alertness value of the human body based on the feature data of the bioelectric signal of the human body received from the feature extraction module 720. In one example, the regression neural network 730 may be obtained by training an existing neural network for regression tasks using a training data set comprising bioelectric signals of the human body and corresponding human body alertness labels. In one example, the regression neural network 730 may comprise an encoder-decoder architecture neural network employing an attention mechanism. Because the bioelectric signal used to estimate the alertness of the human body is a time series signal, it includes contextual information. That is, bioelectric signals at different specific moments within a time window may have different importance for estimating the alertness of the human body. By employing an attention mechanism, the regression neural network 730 can be made to utilize contextual information in input data fully and efficiently when estimating the alertness of the human body.

In one example, the encoder-decoder architecture neural network employing an attention mechanism may comprise one encoder and one decoder. The encoder may comprise at least a first multihead attention module and a first feedforward neural network. The decoder may comprise at least a second multihead attention module, a third multihead attention module, and a second feedforward neural network. The encoder and/or decoder may also comprise one or more addition and normalization modules. The feature data of the bioelectric signal generated by the feature extraction module 720 may be input directly into the first multihead attention module in the encoder and the second multihead attention module in the decoder. Each feedforward neural network may comprise a fully connected neural network comprising one input layer, one or more hidden layers, and one output layer. The output layer of the feedforward neural network may comprise only one neuron.

In one example, the early warning module in the device 700 may judge whether the human body is in a state of fatigue or inattention based on the alertness value of the human body estimated by the regression neural network 730 and send a reminder signal to the human body or other supervisor when the human body is judged to be in a state of fatigue or inattention, e.g., by generating a vibration, issuing an alarm sound, sending a notification message to the supervisor, etc. The early warning module may compare the estimated alertness value to a predetermined threshold. When the estimated alertness value is above the threshold, the human body may be judged to be not fatigued and focused, while when the estimated alertness value is equal to or below the threshold, the human body may be judged to be in poor condition, e.g., fatigued or unfocused. The early warning module may flexibly set thresholds based on different application scenarios.

FIG. 8 illustrates a block diagram of a device 800 for estimating the alertness of the human body according to one example of the present disclosure. The device 800 may be a wearable device (e.g., a helmet, eyewear, watch, etc.) with the function of estimating the alertness of the human body or may be a dedicated device for estimating the alertness of the human body that can be installed on a wearable device, vehicle, etc. As shown in FIG. 8, the device 800 may comprise a memory 810 and at least one processor 820. The processor 820 may be coupled to the memory 810 and configured to perform the method 200 described above with reference to FIG. 2.

For example, the processor 820 can be configured to preprocess a raw bioelectric signal of the human body to at least partially remove noise and artifacts from the raw bioelectric signal; obtain the feature data of the bioelectric signal for estimating the alertness of the human body based on the preprocessed bioelectric signal; and utilize a regression neural network to estimate the alertness value of the human body based on the feature data of the bioelectric signal.

The processor 820 may be a general purpose processor or may be implemented as a combination of computing devices, such as a combination of a DSP and a microprocessor, a plurality of microprocessors, a combination of one or more microprocessors and a DSP core, or any other such configuration. The memory 810 may comprise non-volatile memory for storing computer program code that implements the solution of the present disclosure and various trained parameters of the regression neural network. The memory 810 may also comprise volatile memory for temporary storage of data received during processor execution (e.g., the bioelectric signals of the human body), processed data (e.g., the feature data of bioelectric signals of the human body, the alertness values of the human body), etc.

The various operations described in conjunction with this disclosure may be performed with hardware, software executed by a processor, firmware, or any combination thereof. In one example of the present disclosure, a computer program product for estimating the alertness of the human body may comprise processor-executable computer code for executing the method 200 described above with reference to FIG. 2. In another example of the present disclosure, a computer-readable medium may store computer program code for estimating the alertness of the human body. The computer program code, when executed by the processor, may cause the processor to perform the method 200 described above with reference to FIG. 2. Computer-readable media include both non-transitory computer storage media and communication media. Communication media include any medium that facilitates the transfer of a computer program from one place to another. Any connection may be appropriately referred to as a computer-readable medium. Other examples and implementations are within the scope of the present disclosure.

In addition to the content described in this document, various modifications can be made to the disclosed examples and implementations of the present invention without departing from the scope of the disclosed examples and embodiments of the present invention. Therefore, the description and examples herein should be interpreted as illustrative and not restrictive. The scope of the present invention should only be determined by reference to the claims.

**Claims**

1. A method for estimating the alertness of the human body, comprising:

   obtaining a bioelectric signal from the human body;
   obtaining feature data for estimating the alertness of the human body based on the bioelectric signal; and
   estimating the alertness value of the human body using a regression neural network based on the feature data of the bioelectric signal.

2. The method according to Claim 1, wherein obtaining a bioelectric signal from the human body comprises: obtaining a raw bioelectric signal from the human body; and preprocessing a raw bioelectric signal of the human body to at least partially remove noise and artifacts from the raw bioelectric signal;
   and wherein obtaining the feature data of a bioelectric signal for estimating the alertness of the human body comprises: obtaining feature data for estimating the alertness of the human body based on the preprocessed bioelectric signal.

3. The method according to Claim 2, wherein the raw bioelectric signal comprises at least one of an EEG signal, an EOG signal, and an EMG signal of the human body.

4. The method according to Claim 2, wherein the preprocessing further comprises: Preprocessing a raw bioelectric signal of the human body with a bandpass filter having a bandpass frequency of 1 Hz to 50 Hz.

5. The method according to Claim 1, wherein the feature data comprise at least one of the power spectral density and the differential entropy of a bioelectric signal.

6. The method according to Claim 1, wherein the regression neural network comprises an encoder-decoder architecture neural network employing an attention mechanism.

7. The method according to Claim 6, wherein the encoder-decoder architecture neural network employing an attention mechanism comprises an encoder comprising at least a first multihead attention module and a first feedforward neural network and a decoder comprising a second multihead attention module, a third multihead attention module positioned behind the second multihead attention module, and a second feedforward neural network.

8. The method according to Claim 7, wherein each feedforward neural network of the first and second feedforward neural networks comprises a fully connected neural network comprising one input layer, one or more hidden layers, and one output layer, the output layer comprising only one neuron.

9. The method according to Claim 7, wherein utilizing the regression neural network to estimate the alertness value of the human body comprises:
   inputting the feature data of the bioelectric signal directly into the first and second multihead attention modules.

10. A device for estimating the alertness of the human body, comprising:

    a signal acquisition module for acquiring a bioelectric signal of the human body;
    a feature extraction module for obtaining feature data for estimating the alertness of the human body based on the bioelectric signal; and
    a regression neural network for estimating the alertness value of the human body based on the feature data of the bioelectric signal.

11. A device for estimating the alertness of the human body, comprising:

    a memory;
    a processor coupled to the memory and configured to perform the method according to any one of Claims 1-9.

**12.** A computer program product for estimating the alertness of the human body, comprising processor-executable computer program code for performing the method according to any one of Claims 1-9.

FIG. 1

FIG. 2

FIG. 3

440

430

420 ⌐ h

410    412    414

V    K    Q

FIG. 4

550

540

530

520

510

Q    K    V

FIG. 5

FIG. 6

FIG. 7

FIG. 8

Europäisches
Patentamt

European
Patent Office

Office européen
des brevets

## EUROPEAN SEARCH REPORT

**Application Number**

EP 25 15 7920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAO KAI ET AL: "Multi-Head Self-Attention Enhanced Convolutional Neural Network for Driver Fatigue Detection using EEG Signals", 2023 IEEE INTL CONF ON PARALLEL & DISTRIBUTED PROCESSING WITH APPLICATIONS, BIG DATA & CLOUD COMPUTING, SUSTAINABLE COMPUTING & COMMUNICATIONS, SOCIAL COMPUTING & NETWORKING (ISPA/BDCLOUD/SOCIALCOM/SUSTAINCOM), IEEE, 21 December 2023 (2023-12-21), pages 817-823, XP034586979, DOI: 10.1109/ISPA-BDCLOUD-SOCIALCOM-SUSTAINCOM59178.2023.00139 [retrieved on 2024-04-11] * abstract * * sections II-III; figures 1-5; tables I-III * | 1-12 | INV. A61B5/18 A61B5/369 A61B5/389 A61B5/398 A61B5/00 |
| X | GAO DINGCHENG ET AL: "Driver Vigilance Detection from EEG Signals using Transformer Networks", GLOBECOM 2022 - 2022 IEEE GLOBAL COMMUNICATIONS CONFERENCE, IEEE, 4 December 2022 (2022-12-04), pages 1411-1416, XP034269191, DOI: 10.1109/GLOBECOM48099.2022.10000618 [retrieved on 2023-01-11] * abstract * * Sections II-IV; figures 1-5; table I * | 1-12 | **TECHNICAL FIELDS SEARCHED (IPC)**<br><br>A61B |

-/--

The present search report has been drawn up for all claims

| Place of search | Date of completion of the search | Examiner |
|---|---|---|
| The Hague | 24 April 2025 | Lai, Marco |

Europäisches
Patentamt

European
Patent Office

Office européen
des brevets

# EUROPEAN SEARCH REPORT

**Application Number**

EP 25 15 7920

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHI GUANG ET AL: "A Transformer-Based Spatial-Temporal Sleep Staging Model Through Raw EEG", 2021 INTERNATIONAL CONFERENCE ON HIGH PERFORMANCE BIG DATA AND INTELLIGENT SYSTEMS (HPBD&IS), IEEE, 5 December 2021 (2021-12-05), pages 110-115, XP033995098, DOI: 10.1109/HPBDIS53214.2021.9658439 [retrieved on 2021-12-21] * Sections II-IV; figures 1-3; tables I-III * ----- | 1-12 | |
| X | ELDELE EMADELDEEN ET AL: "An Attention-Based Deep Learning Approach for Sleep Stage Classification With Single-Channel EEG", IEEE TRANSACTIONS ON NEURAL SYSTEMS AND REHABILITATION ENGINEERING, IEEE, USA, vol. 29, 28 April 2021 (2021-04-28), pages 809-818, XP011853209, ISSN: 1534-4320, DOI: 10.1109/TNSRE.2021.3076234 [retrieved on 2021-05-04] * sections II-III; figures 1-7; examples I-VI * * abstract * ----- | 1-12 | TECHNICAL FIELDS SEARCHED (IPC) |

The present search report has been drawn up for all claims

| Place of search | Date of completion of the search | Examiner |
|---|---|---|
| The Hague | 24 April 2025 | Lai, Marco |

EPO FORM 1503 03.82 (P04C01)